# EUROPÄISCHE PATENTSCHRIFT

(11) **EP 2 056 736 B1**
(45) Veröffentlichungstag und Bekanntmachung des Hinweises auf die Patenterteilung: **22.01.2014**
(21) Anmeldenummer: 07801263.0
(22) Anmeldetag: 21.08.2007
(51) Int. Cl.: A61C 8/00

(54) **KIEFERIMPLANTAT MIT EINEM AUS EINEM KERN UND AUS ZUMINDEST EINEM AN DEN KERN RADIAL ANGEFORMTEN GEWINDEGANG BESTEHENDEN, SCHRAUBENFÖRMIGEN VERANKERUNGSTEIL**
JAW IMPLANT WITH A SCREW-SHAPED ANCHORING PART CONSISTING OF A CORE AND AT LEAST A FROM THE CORE RADIALLY EXTENDING THREADED PITCH
IMPLANT DE MÂCHOIRE COMPORTANT UNE PARTIE ANCRAGE HÉLICOÏDALE COMPOSÉE D'UN NOYAU ET D'AU MOINS UN PAS DE VIS RADIAL SUR LE NOYAU

(30) Priorität: 21.08.2006 DE 102006039116; 01.03.2007 DE 102007009935
(43) Veröffentlichungstag der Anmeldung: 13.05.2009
(73) Patentinhaber: Teichmann, Gernot, 40667 Meerbusch (DE)
(72) Erfinder: Teichmann, Gernot, 40667 Meerbusch (DE)
(74) Vertreter: Kreuzkamp, Markus
(86) Internationale Anmeldenummer: PCT/DE2007/001476
(87) Internationale Veröffentlichungsnummer: WO 2008/022629

(56) Entgegenhaltungen:
- EP-A- 1 070 485
- DE-A1- 2 255 916
- DE-A1- 3 136 602
- US-A- 5 975 903

## Beschreibung

Die Erfindung bezieht sich auf ein Kieferimplantat mit einem aus einem Kern und aus zumindest einem an den Kern radial angeformten Gewindegang bestehenden, schraubenförmigen Verankerungsteil.

Derartige Kieferimplantate, auch dentale Implantate genannt, sind in unterschiedlichen Formen vorbekannt. Die bekannten dentalen implantate arbeiten im Bereich der Verankerung im Prinzip wie eine Schraube und können auch selbst gewindeschneidend ausgebildet sein. Genormten Schrauben ähnlich, haben sie stets einen relativ dicken Kern und mehrere Gewindegänge mit relativ geringer Tlefe.

Die EP 1609436 A1 zeigt ein am unteren Ende abgerundetes und sonst durchgehend zylindrisches Verankerungsteil, mit Gewindegängen ähnlich einem Rundgewinde. Die EP 1502558 A1 zeigt ein Verankerungsteil mit einem Spitzgewinde von etwa 70° und am unteren Ende angeformten Schneiden, ähnlich einem Gewindeschneider. Die EP 1563802 A1 zeigt ein konisches Verankerungsteil mit einem Spitzgewinde von etwa 30° und gleichbleibender Gewindetiefe. Am unteren Ende ist dem konischen Kern eine Schneide angeformt Die EP 1527749 A1 zeigt ein leicht konisches Verankerungsteil mit einem Sägezahngewinde von etwa 40° und nach unten zunehmender Gewindetiefe, also mit konischem Kern.

Das Größenverhältnis Außendurchmesser zu Kerndurchmesser des Gewindes liegt beim Stand der Technik bei etwa 1,2 bis 1,8, wobei die größeren Werte nur bei konischen Ausbildungen im unteren Spitzenbereich erreicht werden. Die kleineren Werte liegen hingegen etwa im Normbereich üblicher Schrauben. Die Gewindegänge sind somit relativ kompakt, d. h. mit geringer Tiefe bzw. Höhe, ausgebiklet.

Die vorbekannten Kieferimplantate mit schraubenförmigen Verankerungsteilen sind bestimmt zum Einschrauben in eine von der Mundhöhle aus in den Kieferknochen eingebrachte Bohrung. Das eingeschraubte Kleferimplantat dient zur Aufnahme einer Krone, Brücke oder zur Abstützung einer Prothese oder Teilprothese. Zum einen benötigen die vorbekannten Kieferimplantate zu ihrer sicheren Verankerung eine gewisse Mindestlänge des Verankerungstells und damit des Gewindes, das z. B. beim vorgenannten Stand der Technik aus 7 bis 22 Gewindegängen bestehen kann, um die auf den Zahn oder dergleichen einwirkenden Kaukräfte auf Dauer sicher in den Kieferknochen übertragen zu können. Zum anderen führen die bisherigen schraubenförmigen Kieferimplantate, bedingt durch die oben erläuterte geometrische Ausbildung des Gewindes, zwar zu einer erheblichen radialen, aber nur zu einer geringen axialen Abstützung des Implantats im Kieferknochen. Dies kann über die Nutzungsdauer des Implantats hinweg zu einer Lockerung des Implantats, d.h. zu einem Lösen der festen Verbindung zwischen dem Verankerungsteil und dem Kieferknochen führen.

Aus der DE 22 55 916 A1 ist schon ein schraubenförmiges Verankerungsteil bekannt, das einen Kern und einen an den Kern radial angeformten Schneckengang aufweist, wobei das Verhältnis des Außendurchmessers zum Kerndurchmesser 3:1 beträgt

Die Erfindung geht nun aus von der US 5 975 903 A Dieses Dokument offenbart ein Kieferimplantat, mit einem aus einem Kern und aus zumindest einem an der Kern radial angeformten Gewindegang bestehenden, schraubenförmigen Verankerungsteil, das nach Art einer Schnecke ausgebildet ist, bei der das Größenverhältnis Außendurchmesser zu Kemdurchmesser gleich oder größer als 2 ist und der Schneckengang nur den Bruchteil einer Umdrehung umfasst.

Bei der Verankerung eines langen, schraubenförmiges Implantats nach dem Stand der Technik in einer relativ dünnen Knochenschicht oder Knochenrestschicht können sich aufgrund der Länge des Schraubengangs Schwierigkeiten ergeben: So ist im Oberkieferseitenzahnbereich durch die Ausdehnung der Kleferhöhle die Knochenschicht relativ dünn. Hier muß bisher die Kieferhöhle operativ verkleinert werden, um den gewonnenen Raum mit echtem oder künstlichen Knochen auffüllen zu können, damit eine ausreichend dicke Knochenschicht zur Aufnahme des schraubenförmigen Verankerungsteils entsteht Diese im Fachgebiet als Sinuslift bekannte Operation belastet den Patienten zusätzlich und erhöht generell die Gefahr eines Mißerfolgs einer Kieferimptantation.

In Anbetracht der geschilderten Gegebenheiten liegt der Erfindung das Bestreben zu Grunde, ein Kieferimplantat der gattungsgemäßen Art so auszubilden, dass die Verbindung zwischen seinem Verankerungsteil und dem Kieferknochen auf Dauer sichergestellt ist, selbst wenn aus eine relativ dünne und sogar sehr schmale Knochenschicht vorhanden ist.

Um dies zu erreichen, schlägt die Erfindung vor, dass die Schnecke zweigängig ist und beide Schneckengänge propellerflügelförmig nur mit zwei diametral gegenüberliegenden Teilschneckengängen ausgebildet sind.

Hier durch bietet die Erfindung einen entscheidenden Vorteil: Der Verankerungsteil kann in einem solchen Fall äußerst kurz gehalten werden, indem der Schneckengang nur den Bruchteil einer Umdrehung umtaßt, die Schnecke zweigängig ist und beide Schneckengänge nur mit zwei diametral gegenüberliegenden Teilschneckengängen ausgebildet sind, so dass sich die Form eines zweiflügeligen Propellers ergibt. Bei dieser Form kann der Verankerungsteil in eine relativ dünne und sogar sehr schmale Knochenschicht mit dem Bruchteil einer Umdrehung eingedreht werden. Eine oben erläuterte Operation entfällt, das damit verbundene Risiko ebenfalls. Außerdem wird der Patient geschont, es werden Zeit und Kosten eingespart.

Das genannte Größenverhältnis kann zwischen 2 und 5 betragen, je nach dem, wie groß im jeweiligen individuellen Fall der zur Verfügung stehende Platz im Kieferknochen ist.

Zum praktisch lückenlosen Einbringen des Verankerungsteils in die Öffnung kann weiterhin vorgesehen sein, dass der Schneckengang am unteren Ende über einen zumindest nahezu radialen Bogen aus dem Außendurchmesser in den Kerndurchmesser übergeht.

Zum erleichterten und lückenlosen Eindrehen dienlich ist eine Form, bei welcher der Kern im Querschnitt kreiszylindrisch ausgebildet ist und unten mit einem am Ende abgerundeten Abschnitt etwas aus der Schnecke hervorsteht

Der Außendurchmesser des Verankerungsteils kann über die Länge des Schneckengangs zumindest im Wesentlichen gleich bleiben. Der Schneckengang an sich kann im Querschnitt unterschiedliche Formen aufweisen: Er kann im Prinzip z. B. nach Art eines jeweils sehr flachen Spitzgewindes, Trapezgewindes oder Sägengewindes ausgebildet sein, wobei die Außenspitze oder Außenkante des Schneckengangs zumindest leicht abgerundet sein sollte. Durch die sehr flache, dünne Ausbildung des Schneckengangs ergibt sich hier beim Spitz- oder Sägengewinde, im Verhältnis zu herkömmlichen Gewinden, ein sehr kleiner Spitzenwinkel von etwa 10° bis 20°.

Um dem Verankerungsteil einschließlich Schnecke eine gewisse Stabilität zu verleihen, sollte die Innendicke des Schneckengangs etwa 0,5 bis 2,0 mm und die Außendicke des Schneckengangs maximal 1,0 mm betragen.

>

Die Abmessungen der Schnecke sind den Erfordernissen des jeweiligen Einzelfalls anzupassen. Da eine übliche Befestigungsbohrung im Kern grundsätzlich einen gewissen Raum erfordert, kann der Kemdurchmesser ca. 2 bis 5 mm betragen, wobei ein kleiner Kemdurchmesser von z. B. 3 bis 4 mm aus den genannten Gründen zu bevorzugen ist.

Um den Kieferknochen gut in den Verankerungsteil einwachsen zu lassen, kann vorgesehen werden, dass zumindest ein Teilschneckengang mit zumindest einer Ausnehmung, vorzugsweise aber mit mehreren Ausnehmungen, versehen ist. Eine solche Ausnehmung kann leicht sacklochartig geformt sein oder den Schneckengang axial durchdringen und / oder radial, also seitlich, etwas offen ausgebildet sein.

Weitere besondere Vorteile sind für den Fachmann auch der nachfolgenden Beschreibung in Verbindung mit der Zeichnung entnehmbar, in der ein Ausführungsbeispiel der Erfindung schematisch dargestellt ist.
Fig. 1 zeigt ein Kieferimplantat in eingängiger Ausführung mit nahezu einer Umdrehung in Seitenansicht.
Fig. 2 zeigt das Kieferimplantat nach Fig. 1 in der Draufsicht.
Fig. 3 zeigt ein Kieferimplantat in zweigängiger Ausführung und propellerartiger, zweiflügliger Form in Seitenansicht
Fig. 4 zeigt das Kieferimplantat nach Fig. 3 in Draufsicht mit strichpunktiert angedeutetem Außendurchmesser.
Fig. 5 zeigt die Befestigung eines Zahnersatzes im Oberkiefer mit einer zweigängigen Schnecke im Schnitt.
Fig. 6 zeigt die Befestigung nach Fig. 5 quer zur Schnittebene der Fig. 5 im Schnitt.

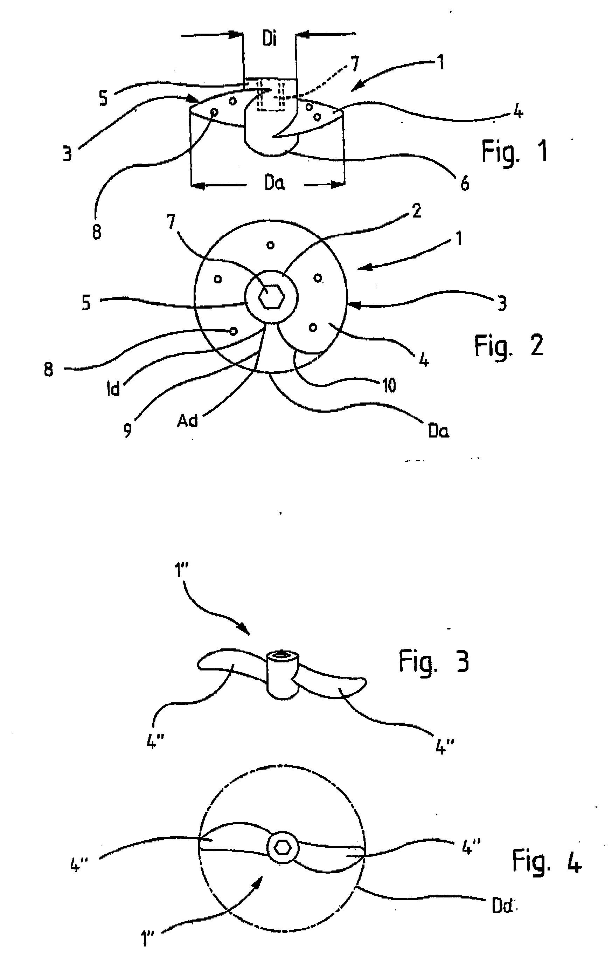

In Fig. 1 ist ein Kieferimplantat in der Seitenansicht schematisch dargestellt. Es umfaßt einen schraubenförmigen Verankerungsteil 1 aus einem zylindrischen im Querschnitt runden Kern 2 und einem daran angeformten Gewindegang in Form einer Schnecke 3. Der Schneckengang 4 ist im Querschnitt wie eine sehr flaches Spitzgewinde ausgebildet, dessen Spitze leicht abgerundet ist. Der gleichblebende Außendurchmesser Da beträgt etwa 10 mm, der Innen- oder Kemdurchmesser Di etwa 4 mm, die Länge etwa 5 mm. Der Kern 2 steht oben und unten axial etwas über den Schneckengang 4 hervor, wobei er oben als Ringstutzen 5 und unten als teilkugelförmige Abrundung 6 endet Im Innern des Kerns 2 ist eine sacklochartige Öffnung 7 eingebracht, um die sich der Ringstutzen 5 erstreckt. Die Öffnung 7 ist im lichten Querschnitt zum Ansatz eines Drehwerkzeugs geeignet ausgebildet, z. B. als Innensechskant oder Innenvielzahn. Der Schneckengang 4 hat am Kern 2 eine Innendicke Id von etwa 1 mm und an der Abrundung eine Außendicke Ad von etwa 0,3 mm, im Mittel also etwa 0,6 mm Der flache Schneckengang 4 ist mit einigen axial durchgehenden Ausnehmungen 8 versehen.

In Fig. 2 ist das Kieferimplantat der Fig. 1 In der Draufsicht dargestellt. Hier wird besonders deutlich, dass der Schneckengang 4 etwas weniger als eine ganze Umdrehung umfaßt. Der Außendurchmesser Da ist strichpunktiert ergänzt. Während das obere Ende des Schneckengangs 4 mit einer geraden radialen Kante 9 zwischen dem Innendurchmesser Di und dem Außendurchmesser Da endet, ist zumindest das untere Ende mit einem Bogen 10 versehen, der sich stetig vom Innendurchmesser Di bis zum Außendurchmesser Da nahezu radial erstreckt. Das Verhältnis Da zu Di betragt hier etwa 2,5. /

Beide Figuren 1 und 2 werden vom Patentanspruch 1 nicht umfasst, zeigen jedoch Merkmale der Unteransprüche.

Fig. 3 zeigt ein Kieferimplantat mit Verankerungsteil1" in zweigängiger Ausführung und propellerartiger, zweiflügeliger Form in der Seitenansicht. Fig. 4 zeigt dieses Verankerungsteil 1" in der Draufsicht mit strichpunktiert angedeutetem Außendurchmesser Da". Hier wird besonders deutlich, dass zwel diametral auf gleicher axialer Höhe angeordnete, kurze Schneckengänge 4" nur einen Bruchteil eines Umfangs abdecken und folglich zur Verankerung nur einen kurzen und extrem schmalen Knochenbereich im Kiefer benötigen. Die übrigen Merkmale entsprechen der zu der Fig. 1 beschriebenen Ausführung.

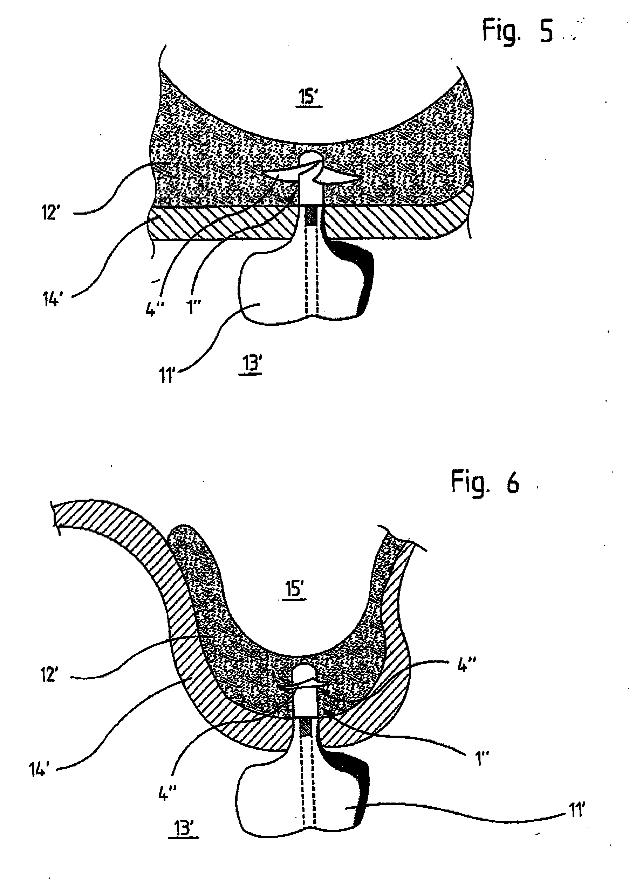

Fig. 5 und 6 zeigen die Befestigung eines Zahnersatzes 11', z. B. einer Krone, im Oberkieferknochen 12', jedoch mit einer zweigängigen Schnecke im Schnitt. Als Verankerungsteil 1" dient hier das zu den Fig. 3 und 4 näher beschriebene. Da die beiden Teilschneckengänge 4" auf der selben axialen Höhe liegen und nur einen Bruchteil des Umfangs einnehmen, ergibt sich eine besonders schmale und niedrige Bauhöhe, so dass nur eine sehr kurzer und vor allem auch sehr schmaler Knochenbereich im Oberkieferknochen 12' zur Verankerung erforderlich ist

### Bezugszeichen

- 1: Verankerungsteil
- 1": Verankerungsteil
- 2: Kern
- 3: Schnecke
- 4: Schneckengang
- 4": Schneckengang
- 5: Ringstutzen
- 6: Abrundung
- 7: Öffnung
- 8: Ausnehmung
- 9: Kante
- 10: Bogen
- 11': Zahnersatz
- 12': Oberkieferknochen
- 13': Mundhöhle
- 14': Zahnfleisch
- 15': Obenkieferhöhle
- Ad: Außendicke
- Da: Außendurchmesser
- Da': Außendurchmesser
- Di: Innendurchmesser
- Id: Innendicke

## Patentansprüche

1. Kieferimplantat mit einem aus einem Kern (2) und aus zumindest einem an den Kern (2) radial angeformten Gewindegang (4") bestehenden, schraubenförmigen Verankerungsteil (1 "), das nach Art einer Schnecke (3) ausgebildet ist, bei der das Größenverhältnis Außendurchmesser (Da) zu Kemdurchmesser (Di) gleich oder größer als 2 ist und der Schneckengang (4") nur den Bruchteil einer Umdrehung umfaßt, **dadurch gekennzeichnet, dass** die Schnecke zweigängig ist und beide Schneckengänge propellerflügelförmig nur mit zwei diametral gegenüberliegenden Teilschneckengängen (4") ausgebildet sind.

2. Kieferimplantat nach Anspruch 1, **dadurch gekennzeichnet, dass** das Größenverhältnis zwischen 2 und 5 beträgt.

3. Kieferimplantat nach Anspruch 1 oder 2, **dadurch gekennzeichnet, dass** der Schneckengang (4) am unteren Ende über einen zumindest nahezu radialen Bogen (10) aus dem Außendurchmesser (Da) in den Kemdurchmesser (Di) übergeht.

4. Kieferimplantat nach einem der Ansprüche 1 bis 3, **dadurch gekennzeichnet, dass** der Kern (2) im Querschnitt kreiszylindrisch ausgebildet ist und unten mit einem am Ende abgerundeten Abschnitt (6) und oben mit einem Ringstutzen (5) etwas aus der Schnecke (3) hervorsteht

5. Kieferimplantat nach einem der Ansprüche 1 bis 4, **dadurch gekennzeichnet, dass** der Außendurchmesser (Da) über die Länge des Schneckengangs (4) zumindest im Wesentlichen gleich bleibt.

6. Kieferimplantat nach einem der Ansprüche 1 bis 5, **dadurch gekennzeichnet, dass** der Schneckengang (4) im Prinzip nach Art eines sehr flachen Spitzgewindes ausgebildet ist.

7. Kieferimplantat nach einem der Ansprüche 1 bis 5, **dadurch gekennzeichnet, dass** der Schneckengang im Prinzip nach Art eines sehr flachen Trapezgewindes oder Sägegewindes ausgebildet ist.

8. Kieferimplantat nach Anspruch 6 oder 7, **dadurch gekennzeichnet, dass** die Außenspitze oder Außenkante des Schneckengangs (4) zumindest leicht abgerundet ist.

9. Kieferimplantat nach einem der Ansprüche 1 bis 8, **dadurch gekennzeichnet, dass** die Innendicke (Id) des Schneckengangs (4) 0,5 bis 2,0 mm beträgt

10. Kleferimplantat nach einem der Ansprüche 1 bis 9, **dadurch gekennzeichnet, dass** die Außendicke (Ad) des Schneckengangs (4) maximal 1,0 mm beträgt.

11. Kieferimplantat nach einem der Ansprüche 1 bis 10, **dadurch gekennzeichnet, dass** der Kerndurchmesser (Di) 2 bis 5 mm beträgt.

12. Kieferimplantat nach einem der Ansprüche 1 bis 11, **dadurch gekennzeichnet, dass** zumindest ein Schneckengang (4) oder Teilschneckengang mit zumindest einer Ausnehmung (8) versehen ist

13. Kieferimplantat nach Anspruch 12, **dadurch gekennzeichnet, dass** die Ausnehmung (8) den Schneckengang (4) axial durchdringt.

14. Kieferimplantat nach Anspruch 12 oder 13, **dadurch gekennzeichnet, dass** die Ausnehmung radial offen ausgebildet ist.

## Claims

1. Jaw implant having an anchoring part (1") which is in the form of a screw and comprises a core (2) and at least one thread pitch (4") which is integrally formed radially on the core (2), which anchoring part is in the form of a worm (3) in which the size ratio of the external diameter (Da) to the core diameter (Di) is equal to or greater than 2 and the worm thread (4") covers only a fraction of one revolution, **characterized in that** the worm has two threads and both worm threads are in the form of a propeller blade with just two diametrically opposite partial worm threads (4").

2. Jaw implant according to Claim 1, **characterized in that** the size ratio is between 2 and 5.

3. Jaw implant according to Claim 1 or 2, **characterized in that** the worm thread (4) changes, at the lower end over an at least approximately radial curve (10), from the external diameter (Da) to the core diameter (Di).

4. Jaw implant according to one of Claims 1 to 3, **characterized in that** the core (2) has a circular-cylindrical cross section, has a section (6) which is rounded at the end and projects somewhat out of the worm (3) at the bottom, and has an annular connecting stub (5) which projects somewhat out of the worm (3) at the top.

5. Jaw implant according to one of Claims 1 to 4, **characterized in that** the external diameter (Da) remains at least essentially constant over the length of the worm thread (4).

6. Jaw implant according to one of Claims 1 to 5, **characterized in that** the worm thread (4) is designed in principle in the form of a very flat V-shaped thread.

7. Jaw implant according to one of Claims 1 to 5, **characterized in that** the worm thread is designed in principle in the form of a very flat trapezoidal thread or sawtooth thread.

8. Jaw implant according to Claim 6 or 7, **characterized in that** the outer tip or outer edge of the worm thread (4) is at least slightly rounded.

9. Jaw implant according to one of Claims 1 to 8, **characterized in that** the inner thickness (Id) of the worm thread (4) is 0.5 to 2.0 mm.

10. Jaw implant according to one of claims 1 to 9, **characterized in that** the outer thickness (Ad) of the worm thread (4) is at most 1.0 mm.

11. Jaw implant according to one of Claims 1 to 10, **characterized in that** the core diameter (Di) is 2 to 5 mm.

12. Jaw implant according to one of Claims 1 to 11, **characterized in that** at least one worm thread (4) or partial worm thread is provided with at least one recess (8).

13. Jaw implant according to Claim 12, **characterized in that** the recess (8) passes axially through the worm thread (4).

14. Jaw implant according to Claim 12 or 13, **characterized in that** the recess is open radially.

## Revendications

1. Implant de mâchoire comportant une partie d'ancrage hélicoïdale (1") composée d'un noyau (2) et d'au moins un pas de vis radial (4") formé sur le noyau (2), qui est réalisée à la manière d'une vis sans fin (3), dans laquelle le rapport dimensionnel du diamètre extérieur (Da) au diamètre du noyau (Di) est égal ou supérieur à 2 et le pas de vis (4") ne comprend que la fraction d'une révolution, **caractérisé en ce que** la vis sans fin est à filet double et les deux filets de vis sont réalisés en forme de pales d'hélice uniquement avec deux filets de vis partiels (4") diamétralement opposés l'un à l'autre.

2. Implant de mâchoire selon la revendication 1, **caractérisé en ce que** le rapport dimensionnel est compris entre 2 et 5.

3. Implant de mâchoire selon la revendication 1 ou 2, **caractérisé en ce que** le filet de vis (4) passe du diamètre extérieur (Da) au diamètre de noyau (Di) à l'extrémité inférieure par un arc au moins presque radial (10).

4. Implant de mâchoire selon l'une quelconque des revendications 1 à 3, **caractérisé en ce que** le noyau (2) présente une section transversale cylindrique ronde et est légèrement saillant hors de la vis sans fin (3) dans le bas avec une partie (6) arrondie à l'extrémité et en haut avec un appui annulaire (5).

5. Implant de mâchoire selon l'une quelconque des revendications 1 à 4, **caractérisé en ce que** le diamètre extérieur (Da) reste au moins essentiellement constant sur la longueur du filet de vis (4).

6. Implant de mâchoire selon l'une quelconque des revendications 1 à 5, **caractérisé en ce que** le filet de vis (4) est réalisé en principe à la manière d'un filet triangulaire très plat.

7. Implant de mâchoire selon l'une quelconque des revendications 1 à 5, **caractérisé en ce que** le filet de vis est réalisé en principe à la manière d'un filet trapézoïdal ou d'un filet de scie très plat.

8. Implant de mâchoire selon la revendication 6 ou 7, **caractérisé en ce que** la pointe extérieure ou l'arête extérieure du filet de vis (4) est au moins légèrement arrondie.

9. Implant de mâchoire selon l'une quelconque des revendications 1 à 8, **caractérisé en ce que** l'épaisseur intérieure (Id) du filet de vis (4) vaut 0,5 à 2,0 mm.

10. Implant de mâchoire selon l'une quelconque des revendications 1 à 9, **caractérisé en ce que** l'épaisseur extérieure (Ad) du filet de vis (4) vaut au maximum 1,0 mm.

11. Implant de mâchoire selon l'une quelconque des revendications 1 à 10, **caractérisé en ce que** le diamètre de noyau (Di) vaut 2 à 5 mm.

12. Implant de mâchoire selon l'une quelconque des revendications 1 à 11, **caractérisé en ce qu'**au moins un filet de vis (4) ou un filet de vis partiel est doté d'au moins un évidement (8).

13. Implant de mâchoire selon la revendication 12, **caractérisé en ce que** l'évidement (8) traverse axialement le filet de vis (4).

14. Implant de mâchoire selon la revendication 12 ou 13, **caractérisé en ce que** l' évidement est réalisé sous forme ouverte radialement.
